# EUROPEAN PATENT SPECIFICATION

(11) **EP 1 354 942 B1**
(45) Date of publication and mention of the grant of the patent: **30.01.2008**
(21) Application number: 03008738.1
(22) Date of filing: 16.04.2003
(51) Int. Cl.: C12N 5/06

(54) **Induction of insulin-producing cells**
Induktion von Insulin-produzierende Zellen
Induction de cellules produisant de l'insuline

(30) Priority: 17.04.2002 JP 2002115064
(43) Date of publication of application: 22.10.2003
(73) Proprietor: JCR PHARMACEUTICALS Co., LTD., Ashiya, Hyogo 659-0021 (JP); Seino, Susumu, Chiba-shi, Chiba 260-0852 (JP)
(72) Inventor: Seino, Susumu, Chiba-shi, Chiba 260-0852 (JP); Ishizuka, Nobuko, Chiba-shi, Chiba 261-0001 (JP); Okuno, Masaaki, Chiba-shi, Chiba 260-0855 (JP)
(74) Representative: Reinhard - Skuhra - Weise & Partner

(56) References cited:
- WO-A-00/72885
- WO-A-01/39784
- YANG LIJUN ET AL: "In vitro transdifferentiation of adult hepatic stem cells into pancreatic endocrine cells." BLOOD, vol. 98, no. 11 Part 1, 16 November 2001 (2001-11-16), page 548a XP009013671 43rd Annual Meeting of the American Society of Hematology, Part 1;Orlando, Florida, USA; December 07-11, 2001, November 16, 2001 ISSN: 0006-4971
- FERBER SARAH ET AL: "Pancreatic and duodenal homeobox gene 1 induces expression of insulin genes in liver and ameliorates streptozotocin-induced hyperglycemia" NATURE MEDICINE, NATURE AMERICA, NEW YORK, US, vol. 6, no. 5, May 2000 (2000-05), pages 568-572, XP002154420 ISSN: 1078-8956
- LUMELSKY NADYA ET AL: "Differentiation of embryonic stem cells to insulin-secreting structures similar to pancreatic islets" SCIENCE, AMERICAN ASSOCIATION FOR THE ADVANCEMENT OF SCIENCE,, US, vol. 292, no. 5520, 2001, pages 1389-1394, XP002183377 ISSN: 0036-8075

## Description

### FIELD OF THE INVENTION

The present invention relates to a method for preparation of insulin-producing cells from non-insulin-producing cells, and in particular to preparation of insulin-producing cells from hepatocytes.

### BACKGROUND OF THE INVENTION

Since its discovery, insulin has been generally used for the treatment of diabetic patients with absolute insulin deficiency. However, while normal pancreatic β-cells continuously adjust insulin secretion in response to varying blood glucose levels, exogenous insulin administration cannot confine the levels of blood glucose within a physiological range, within which the development of various diabetic complications could be prevented. Although transplantation of pancreas or pancreatic islets can achieve normoglycemia in absolute insulin insufficiency [Robertson RP et al., Diabetes Care 23:112-116 (2000)], the shortage of transplantable pancreas or pancreatic islets, in particular, makes this approach impractical. For this reason, transplantation of pancreatic β-cells or islets generated from stem cells has become more promising therapeutic approach to achieving normoglycemia [Soria B et al., Diabetes 49:157-162 (2000), Lumelsky N et al., Science 292:1389-1394 (2001), Assady S et al., Diabetes 50:1691-1697 (2001)]. The first step of a cell therapy for diabetes mellitus is to generate insulin-secreting cells that are implantable into the patients.

While the establishment of embryonic stem cell (ES cell) lines has provided a useful system to examine the mechanisms of differentiation of stem cells into a variety of types of cells, there are daunting obstacles to the clinical use of ES cells. Allotransplantation of human ES cell derivatives into patients generally would elicit an immune response similar to that is elicited to transplanted pancreatic islets or transplanted pancreas [Odorico JS et al., Stem Cells 19:193-204 (2001)]. In addition, transplantation of ES cell derivatives into human recipients might result in the formation of ES cell-derived tumors [Odorico JS et al., Stem Cells 19:193-204 (2001)]. Ethical issues also would arise in acquiring human ES cell derivatives [McLaren A et al., Nature 414:129-131 (2001)].

Another approach to the replacement of pancreatic β-cells is the transplantation of autologous β-cells that have been generated *ex vivo* from the patients' own stem or multipotent progenitor cells. Adult tissues' stem cells or multipotent progenitor cells were recently reported that are capable of differentiation into various types of cells [Clarke D et al., Curr Opin Genet Dev 11:575-580 (2001)]. Pancreatic islet-like structures have been formed *in vitro* from pancreatic stem cells of humans and adult mice [Bonner-Weir S et al., Proc Natl Acad Sci USA 97:7999-8004 (2000), Ramiya VK et al., Nat Med 6:278-282 (2000), Zulewski H et al., Diabetes 50:521-533 (2001)]. It has also been reported that transfer of the PDX-1 gene into the liver induces insulin-producing cells *in vivo* [Ferber S et al., Nat Med 6:568-572 (2000)]. These findings demonstrate the presence in adult tissues of multipotent progenitor cells capable of differentiating into insulin-producing cells.

Hepatocytes possess several characteristics in common with β-cells. For example, both hepatocytes and β-cells are of endoderm origin [Well JM et al., Annu Rev Cell Dev Biol 15:393-410 (1999)], and the glucose transporter GLUT2 and glucokinase, which are required in glucose sensing, are present in both of hepatocytes and β-cells. In addition, the HNF transcription network, which is important in the development of both hepatocytes and β-cells, controls the expression of genes involved in glucose metabolism [Bell GI et al., Nature 414:788-791 (2001)]. These findings suggest the possibility of generation of cells with the phenotype of pancreatic β-cell from hepatic progenitor cells.

In addition to the synthesis of insulin, there are other essential features of pancreatic β-cells, including glucose-responsiveness, electrical excitability and regulated exocytosis. The K_{ATP} channel in pancreatic *β*-cells, as a metabolic sensor, couples glucose signaling to electrical activity [Seino S, Annu Rev Physiol 61:337-362 (1999)]. Closure of the K_{ATP} channels depolarizes the β-cell membrane and opens the voltage-dependent calcium channels (VDCCs), allowing calcium influx that triggers exocytosis [Wollheim CB et al., Diabetes Rev 4:276-297 (1996)].

A recent study has shown that both the insulin 1 and 2 genes are expressed in mouse hepatic progenitor cells during long-term culture in a medium containing nicotinamide [Suzuki A et al., J Cell Biol 156:173-184 (2002)]. However, there has been no report about the expression of genes associated with other essential features of β-cells, i.e., glucose-responsiveness, electrical excitability and regulated exocytosis.

As the liver and pancreas are both of endodermal origin, derived from the upper primitive foregut [Well JM et al., Annu Rev Cell Dev Biol 15:393-410 (1999)], interconversion between liver and pancreas cells is possible [Ferber S et al., Nat Med 6:568-572 (2000), Shen CN et al., Nat Cell Biol 2:879-887 (2000)]. Indeed, adenovirus-mediated transfer of the PDX-1 gene to mouse liver *in vivo* induced transdifferentiation of a hepatocyte subpopulation to a pancreatic β-cell phenotype [Ferber S et al., Nat Med 6:568-572 (2000)]. However, there has been no report of *in vitro* transdifferentiation of mouse hepatic cells to a pancreatic β-cell phenotype.

The objective of the present invention is to provide insulin-producing cells from non-insulin-producing fetal hepatocytes, and in particular to provide cells expressing not only insulin but also other genes characteristic of β-cells, in particular the SUR1 (a subunit of the ATP-sensitive K⁺ channel) gene, the α₁1.3 (a subunit of the L-type voltage-dependent calcium channel) gene, and more preferably also the prohormone convertase PC 1/3 gene.

### SUMMARY OF THE INVENTION

The present inventors cultured mouse fetal hepatocytes under conditions in which primitive hepatic progenitor cells differentiate into hepatocytes or biliary epithelial cells [Suzuki A et al., Hepatology 32:1230-1239 (2000)]. As a result, it was found that cells were derived that were clearly expressing both of the insulin 1 and 2 genes by culturing in a culture system containing high concentration of nicotinamide. It was found that, by bringing about expression of NeuroD (also called BETA2), which is a transcription factor required in neuronal differentiation, in non-human fetal hepatocytes and culturing the cells in the above-mentioned culturing system, cells are obtained which not only have increased expression levels of SUR1 and α₁1.3 but also express PC1/3, a principal prohormone convertase involved in regulated secretion in neuroendocrine cells [Steiner DF, Curr Opin Chem Biol 2:31-39 (1998)].

Thus, the present invention provides a method for preparation of insulin-producing cells from non-insulin-producing cells, wherein the non-insulin-producing cells are non-human mammalian fetal hepatocytes, and wherein the method comprises culturing the non-human mammalian fetal hepatocytes with 1-50 mmol/L of nicotinamide and concurrently bringing about expression of the NeuroD gene in the non-human mammalian fetal hepatocytes. By this method, such cells are obtained that express the insulin 1 and 2 genes and have increased expression levels of SUR1 and α₁1.3, which are essential to the regulation of insulin secretion. Incorporation of the NeuroD gene, in particular, give such cells that further have increased expression levels of PC1/3, which is necessary for the production of mature insulin.

The above-mentioned method is designed to cause transdifferentiation of hepatic progenitor cells included in non-human mammalian fetal hepatocytes into insulin-producing cells. Therefore, the present invention also provides a method for preparation of insulin-producing cells from non-insulin-producing cells, wherein the non-insulin-producing cells are mammalian hepatic progenitor cells, and wherein the method comprises culturing the mammalian hepatic progenitor cells with 1-50 mmol/L of nicotinamide and concurrently bringing about expression of the PDX-1 gene or the NeuroD gene in the mammalian hepatic progenitor cells.

Further, the present invention provides a method for causing transdifferentiation of non-fetal mammalian hepatic progenitor cells into insulin-producing cells, wherein the method comprises culturing the mammalian hepatic progenitor cells with 1-50 mmol/L of nicotinamide and bringing about expression of the NeuroD gene in the mammalian hepatic progenitor cells.

The present invention thus provides a means for preparation of insulin-producing cells using other cells than pancreatic β-cells. In particular, the present invention enables to prepare cells with the phenotype of pancreatic β-cells using somatic stem/progenitor cells that may be present in the adult liver. Further, without use of ES cells in the present invention, an advantage of the present invention is that it can avoid the problem of tumor development that could be brought about by the use of ES cells.

Furthermore, the present invention provides non-human mammalian fetal hepatocyte-derived, NeuroD gene-expressing insuling-producing cells prepared by a method comprising culturing non-human mammalian fetal hepatocytes with 1-50 mmol/L of nicotinamide and concurrently bringing about expression of the NeuroD gene in the non-human mammalian fetal hepatocytes, wherein the expression of the gene is achieved through introduction of the gene into the non-human mammalian fetal hepatocytes.

In a further embodiment, the invention provides non-fetal mammalian hepatic progenitor cell-derived, NeuroD gene expressing insulin-producing cells prepared by a method comprising culturing non-fetal mammalian hepatic progenitor cells with 1-50 mmol/L of nicotinamide and concurrently bringing about expression of the NeuroD gene in the mammalian hepatic progenitor cells, wherein the expression of the gene is achieved through introduction of the gene into the mammalian hepatic progenitor cells.

In a preferred embodiment, these non-fetal mammalian hepatic progenitor cells are non-human.

### BRIEF DESCRIPTION OF THE FIGURES

Figure 1 shows the comparison of the expression of insulin gene with and without supplementation with nicotinamide.
Figure 2 shows the result of reaction for detection of insulin in the cells cultured without supplementation with nicotinamide.
Figure 3 shows the result of reaction for detection of insulin in the cells cultured with supplementation with nicotinamide.
Figure 4 shows the comparison of the expression of genes in the cultured cells. "no cDNA" = no template for PCR, "MIN6" = pancreatic β-cell line
Figure 5 illustrates a map of pCl-neo vector.
Figure 6 illustrates a map of cosmid vector pAxcw.

### DETAILED DESCRIPTION OF THE INVENTION

In the present invention, the term non-human "fetal hepatocytes" means the cells forming a non-human fetal lever. The term "hepatic progenitor cells" means such cells that are included in the population of the cells forming a liver and that have the potential to develop into cells with other phenotypes in addition to that of hepatocytes.

In the present invention, non-human "fetal hepatocytes" or "hepatic progenitor cells" employed may be such cells from any of mammalian animals including human (for the hepatic progenitor cells) and non-human mammalian animals. Examples of non-human mammalian animals include, but are not limited to, rodents such as mouse and the like, bovine, horse, pig, goat, sheep, dog, cat and the like, and any animal may be chosen insofar as it is of the same species as the animal that is to receive implantation of the insulin-producing cells prepared according to the present invention.

In the method of the present invention, the concentration of nicotinamide in the culture medium is about 1-50 mmol/L, preferably 2-30 mmol/L, and more preferably 5-20 mmol/L.

In the present invention, the method for bringing about expression of the NeuroD gene in non-human fetal hepatocytes or hepatic progenitor cells is not limited to a particular method. For example, it may be achieved by introducing the NeuroD gene into the cells. Introduction of the NeuroD gene into non-human fetal hepatocytes may be performed, for example but not limited to, through infection of the non-human fetal hepatocytes with NeuroD-recombinant adenovirus. Any other method may be employed for introduction of the NeuroD gene, provided that the NeuroD gene thus introduced is expressed in the non-human fetal hepatocytes.

### EXAMPLES

### Preparation of non-human Fetal Hepatocytes

For preparing non-human fetal hepatocytes, the liver of fetal ICR mice (embryonic day 13.5) was excised and treated with 0.1 % trypsin-phosphate-buffered saline for 5 min at 37 °C. A culture medium solution then was added, and the mixture was filtered through a nylon membrane (#200). The cell suspension thus obtained was centrifuged at 50g for 5 min. The cell pellet was suspended in a medium consisting of 1:1 DMEM/F12 (Sigma D6421) supplemented with 10 % fetal bovine serum, L-glutamine (2 mmol/L), β-mercaptoethanol (β-ME), (50 µmol/L), penicillin (100,000 U/L) and streptomycin (100 mg/L). The formula of the 1:1 DMEM/F12 (Dulbecco's Modified Eagle's Medium/Ham's Nutrient Mixture F-12: Sigma D6421) was as follows (in g/L): Ca₂Cl₂/2H₂O (0.1545), CuSO₄/5H₂O (0.0000013), Fe(NO₃)₃/9H₂O (0.00005). FeSO₄/7H₂O (0.000417), MgCl₂/6H₂O (0.0612), MgSO₄ (0.04884), KCl (0.3118), NaHCO₃ (1.2), NaCI (6.996), Na₂HPO₄ (0.07102), NaH₂PO₄ (0.0543), ZnSO₄/7H₂O (0.000432), L-Alanine (0.0045), L-arginine hydrochloride (0.1475), L-asparagine/H₂O (0.0075), L-aspartic acid (0.00665), L-cysteine hydrochloride/H₂O (0.01756), cystine dihydrochloride (0.03129), L-glutamic acid (0.00735), glycine (0.01875), L-histidine hydrochloride/H₂O (0.03148), L-isoleucine (0.05447), L-leucine (0.05905), L-lysine hydrochloride (0.09125), L-methionine (0.01724), L-phenylalanine (0.03578), L-proline (0.01725), L-serine (0.02625), L-threonine (0.05345), L-tryptophan (0.00902), L-tyrosine 2Na/2H₂O (0.05579), L-valine (0.05285), biotin (0.0000035), choline chloride (0.00898), folic acid (0.00265), myoinositol (0.0126), nicotinamide (0.00202 = 0.0165 mmol), D-pantothenic acid/1/2Ca (0.00224), pyridoxine hydrochloride (0.002031), riboflavin (0.000219), thiamine hydrochloride (0.00217), vitamin B12 (0.00068), D-glucose (3.15), HEPES (3.5745), hypoxanthine (0.0021), linoleic acid (0.000042), phenol red-Na (0.00863), putrecine dihydrochloride (0.000081), sodium pyruvate (0.055), DL-thioctic acid (0.000105), thymidine (0.000365).

For a recombinant adenovirus infection experiment, the cells were cultured in the above medium supplemented with insulin (172 nmol/L), dexamethasone (100 nmol/L), epidermal growth factor (EGF) (20µg/L) and nicotinamide (10 mmol/L=1.2g/L). Pelletized cells were suspended in the culture medium and 6 × 10⁶ cells were plated on 3.5-cm petri dishes coated with collagen type I, and incubated in 5 % CO₂, 95 % air at 37 °C.

### [Infection with Recombinant Adenovirus]

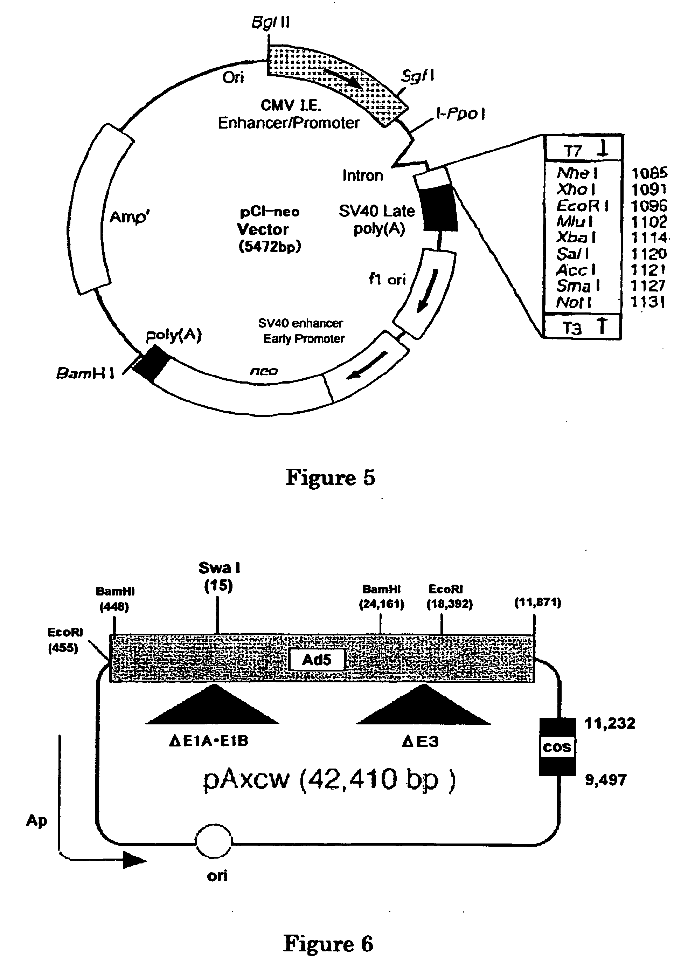

Using an adenovirus expression vector kit (Product Code 6150, TAKARA) and according to the manufacturer's instructions, mouse PDX-1 recombinant adenovirus (AdCMVPDX-1) and human NeuroD-recombinant adenovirus (AdCMVNeuroD) were constructed which had, under the cytomegalovirus (CMV) immediate-early enhancer/promoter [of the cloning vector pCI-neo (GenBank Accession No. U47120: Promega Catalogue No. E1841) shown in Fig. 5], the coding region DNA of mouse PDX-1 and human NeuroD, respectively.

The PDX-1 was cloned by PCR using the cDNAs of a cultured pancreatic β-cell line (MIN6) as templates and with reference to the nucleotide sequence assigned a GenBank Accession No. NM_008814. The primers employed were as follows.
Sense primer: CTAAGGCCTGGCTTGTAGCT (SEQ ID NO:23)
Antisense primer: CGGCTATCCAACTGGCTCTC (SEQ ID NO:24)

By this PCR, a DNA encoding PDX-1 (SEQ ID NO:25) (coding region: nucleotides 59-914) was obtained, which then was incorporated into adenovirus.

A DNA (SEQ ID NO:27, coding region: nucleotides 13-1083) containing the human NeuroD gene's entire coding region, which had been disclosed with the GenBank Accession No. AF045152 (SEQ ID NO:26) (coding region: nucleotides 103-1173), was obtained and incorporated into adenovirus.

For construction of recombinant adenoviruses, cosmid vector pAxcw was employed, which was included in the adenovirus expression vector kit (TAKARA). The structure of this cosmid vector is shown in Fig. 6. This vector has a SwaI site for insertion of exogenous genes.

At the Swal cloning site of the vector pAxcw were inserted, in a conventional manner, the cytomegalovirus immediate-early enhancer/promoter and the PDX-1 or the NeuroD obtained above. Briefly, an expression unit (blunt ended) was prepared containing the cytomegalovirus immediate-early enhancer/promoter and the coding region of the PDX-1 or NeuroD, then added to the cosmid vector pAxcw completely digested with Swal, precipitated with ethanol, and subjected to ligation reaction. The recombinant cosmid vector thus obtained was λ-packaged, with which *E. coli* cells then were infected. Cosmid clones were digested with Clal to cut out the insert for check, and the clones having the inserted expression unit were selected. Each selected cosmid clone, with its circular structure intact, was λ-packaged, with which *E. coli* cells then were infected. By culturing the *E. coli* cells, the cosmid clone was prepared on a large scale. The cosmid DNA was mixed with the restriction enzyme-treated DNA-TPC (adenovirus genomic DNA-terminal protein complex), which was included in the kit, and used to transfect cultured 293 cells by the calcium phosphate method. The cells were cultured and recombinant adenoviruses were collected. 293 cells and HeLa cells were infected with each of the collected recombinant adenovirus samples, and only those samples were selected that killed 293 cells but caused no denaturation in HeLa cells. After confirming the structure of these recombinant viruses', culture of 293 cells infected with each of these viruses allowed to collect the intended recombinant adenoviruses AdCMVPDX-1 and AdCMVNeuroD.

As a control, a recombinant adenovirus was prepared in the same manner using the control cosmid pAxCAiLacZ included in the kit.

After a four-day culture, the isolated mouse fetal hepatocytes were infected with each of the recombinant adenoviruses, AdCMVPDX-1 and AdCMVNeuroD or with the control virus at multiplicity of infections (moi) within a range of 1-200, and cultured for two days for determination of the expressions of various genes. The expression of PDX-1 and NeuroD was confirmed by immunoblot analysis using antibodies specific for human PDX-1 and mouse NeuroD (Santa Cruz Biotechnology, Santa Cruz CA), respectively.

Total RNAs were isolated from mouse fetal hepatocytes using a kit, RNeasy mini (Qiagen, Tokyo). RNA samples were treated with DNaseI (Invitrogen, Carlsbad, CA). cDNAs were prepared using 3 µg of the total RNAs and 25 pmol of pd(N)6 primer (Invitrogen) in a 20-µL solution. With reference to the sequences registered in GenBank, PCR primers were designed for each of the DNAs to be amplified so that the amplified regions spanned an intron in the gene except for the Kir6.2 gene, which has no intron in the protein-coding region (TABLE 1).

**TABLE 1**

| Primer Sequences and PCR Conditions | | | | | |
|---|---|---|---|---|---|
| | Primer sequences | Size of | GenBank | PCR | |
| | Sense primers | product | accession | Temp. | |
| | Antisense primers | (bp) | No. | (°C) | Cycles |
| Insulin 1 | TAGTGACCAGCTATAATCAGAG(*1) | 289 | X04725 | 62 | 35 |
| | ACGCCAAGGTCTGAAGGTCC(*2) | | | | |
| Insulin 2 | CCCTGCTGGCCCTGCTCTT(*3) | 213 | X04724 | 62 | 35 |
| | AGGTCTGAAGGTCACCTGCT(*4) | | | | |
| GLUT2 | TGAGTTCCTTCCAGTTCG(*5) | 183 | NM_031197 | 62 | 30 |
| | AGGGAGCTGGTGTTGTGTA(*6) | | | | |
| GK | CAGAAGGGAACAACATCGTG(*7) | 358 | L41631 | 58 | 35 |
| | CCGCCAATGATCTTTTCGTA(*8) | | | | |
| HK I | TGAAGAAGAAGCTGCGGTCA(*9) | 440 | J05277 | 60 | 28 |
| | TACATCGTGACCCACACAGT(*10) | | | | |
| Kir6.2 | TAGGCCAAGCCAGTGTAGTG(*11) | 248 | U73626 | 61 | 35 |
| | GTGGTGAACACATCCTGCAG(*12) | | | | |
| SUR1 | GGATGAATGCCTTCATCAAG(*13) | 309 | AF037278 | 60 | 35 |
| | GGAAGACGTGGTTCTCCTTC(*14) | | | | |
| α₁1.3 | CTTCGTCATCGTCACCTTCCA(*15) | 254 | M57975 | 60 | 33 |
| | TGAACATCTTGGACTGCTCA(*16) | | | | |
| PC1/3 | ATGGAGCAAAGAGGTTGGAC(*17) | 419 | M58589 | 60 | 35 |
| | GCTGCAGTCATTCTGGTATC(*18) | | | | |
| PC2 | TCGCCAAGTTGCAGCAGAAC(*19) | 314 | M55669. | 60 | 35 |
| | CTTCGGCCACGTTCAAGTCTA(*20) | | | | |
| α-Tubulin | CAGATCGGCAATGCCTGCTG(*21) | 410 | NM_011653 | 62 | 22 |
| | GAGGTGAAGCCAGAGCCAGT(*22) | | | | |

| | | | | | |
|---|---|---|---|---|---|
| Temp., annealing temperature; GK, glucokinase; HK I, hexokinase I *1-22, sequence numbers 1-22 | | | | | |

Alfa-tubulin primers were used to show that each sample contained an equivalent amount of mRNAs. The PCR conditions were as follows: denaturation at 94 °C for 15 sec, annealing for 30 sec, extension at 72 °C for 45 sec. The annealing temperature and the number of cycles were as shown in TABLE 1.

### [Immunohistochemistry]

Fetal hepatocytes were fixed with 4 % paraformaldehyde in 0.1 M phosphate buffer and stained by direct immunoperoxidase method using a guinea pig anti-pig insulin antibody (Zymed Laboratories, South San Francisco, CA) and a peroxidase-labeled pig anti-guinea pig IgG (DAKO Japan, Kyoto).

### Induction of Insulin-Producing Cells from Mouse Fetal Hepatocytes

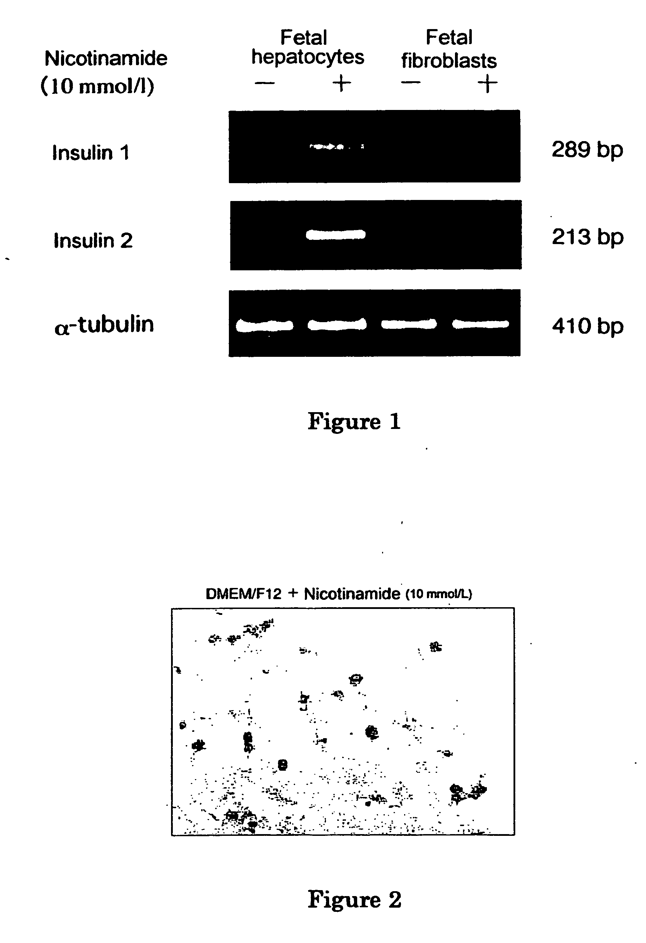

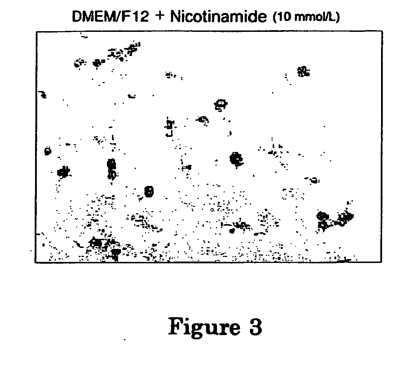

Both insulin 1 and 2 genes were clearly expressed under the culture conditions used, as determined by RT-PCR analysis. It was assumed that some of the supplements (nicotinamide, EGF, insulin or dexamethasone) to the culture medium were responsible for the expression of the insulin genes in the hepatocytes. Each of these supplements was separately added to, the DMEM/F12 containing 10 % FBS, and effects on insulin expression was evaluated in thus formed different media. When cultured in a medium not supplemented with nicotinamide (containing just 0.0165 mmol/L of nicotinamide), the mouse fetal hepatocytes either din not express the insulin 1 or 2 gene at all, or did expressed them but only very poorly (Fig. 1). In contrast, when cultured in a medium supplemented with 10 mmol/L of nicotinamide, the expression of the insulin 1 and 2 genes was both markedly induced in the fetal hepatocytes (Fig. 1). As a negative control, mouse embryonic fibroblasts (MEF) were prepared from fetal limbs (ED13.5), and cultured under the same conditions. Both of the insulin 1 and 2 genes were expressed in the MEF cultured in the medium supplemented with nicotinamide (Fig. 1). The other supplements (i,e., EGF, insulin and dexamethasone) either had no effect or had only a marginal effect on the induction of insulin 1 and 2 gene expression. Immunohistochemistry using anti-insulin antibody was employed to confirm that a high-concentration of nicotinamide was requited for the differentiation of mouse fetal hepatocytes into insulin-producing cells. Culturing in a medium supplemented with nicotinamide led to generation of insulin-positive cells (Fig. 2; not supplemented with nicotinamide, Fig. 3; supplemented with nicotinamide). These results demonstrates that mouse fetal hepatocytes contain progenitor cells capable of differentiating into insulin-producing cells, and that high concentrations of nicotinamide is essential in inducing differentiation.

### [Induction by PDX-1 of Expression of Genes Associated with the β-Cell Phenotype]

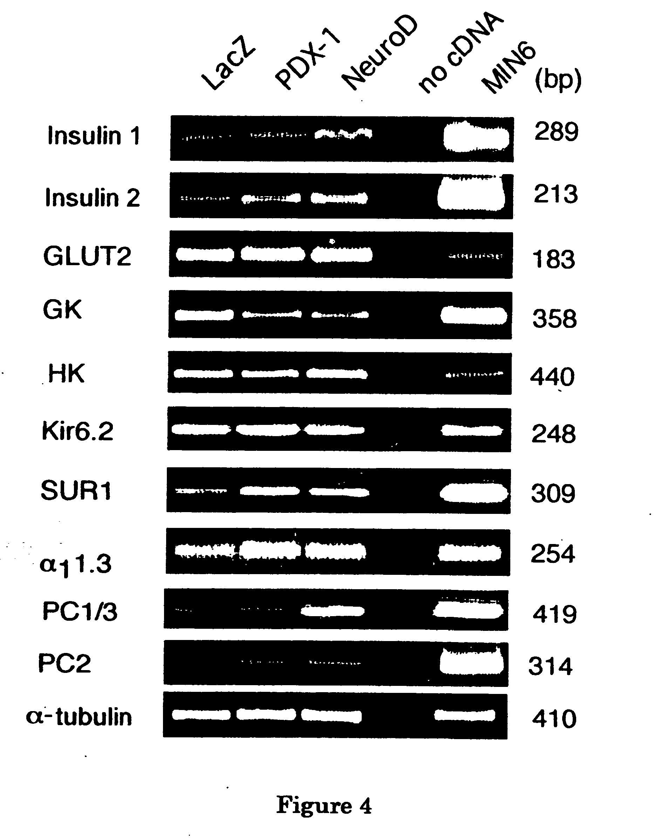

Using an adenovirus-mediated gene transfer system, PDX-1 was introduced into mouse fetal hepatocytes, and expression of various genes was examined 48 hours after infection (Fig. 4). When fetal hepatocytes were cultured in the medium of the culture system for hepatic progenitor cells, both of the insulin 1 and 2 genes were expressed in mock (LacZ)-infected fetal hepatocytes (i.e., control fetal hepatocytes). However, PDX-1 did not significantly increase the expression levels of the insulin genes.

The glucose sensing apparatus, consisting mainly of a specific isoform of glucose transporter, GLUT2 [Thorens B, Mol Membr Biol 18:265-273 (2001)] and type IV hexokinase, glucokinase (GK) [Matschinsky FM et al., Diabetes 47:307-315 (1998)], is common to pancreatic β-cells and hepatocytes. GLUT2 and type I hexokinase (HK1) were expressed in the control fetal hepatocytes, and overexpression of PDX-1 had no effect on their expression (Fig. 4). Glucokinase was markedly expressed in the fetal hepatocytes under the employed culture conditions.

Since K_{ATP} channels and VDCCs are both critical molecules in glucose-responsiveness, electrical activity and regulated exocytosis, all of which characterize the pancreatic β-cell phenotype [Seino S, Annu Rev Physiol 61:337-362 (1999), Ashcroft FM et al., Prog Biophys Mol Biol 54:87-143 (1989)], the present inventors evaluated the expression of the ion channel subunits (Fig. 4). The β-cell K_{ATP} channel comprises two different subunits: the pore-forming Kir6.2 subunit, of the inward rectifier K⁺ channel family, and the regulatory SUR1 subunit, a receptor of the sulfonylureas widely used in the treatment of type 2 diabetes [Seino S, Annu Rev Physiol 61:337-362 (1999)]. Both of the Kir6.2 and SUR1 genes were expressed in the control fetal hepatocytes. The expression of the Kir6.2 gene was not affected by PDX-1, but that of the SUR1 gene was increased significantly by overexpression of PDX-1 (Fig. 4). The expression of the α₁1.3 subunit gene, which is the pore-forming subunit of L-type VDCCs in pancreatic β-cells, was detected in the control fetal hepatocytes, and its expression was increased by overexpression of PDX-1 (Fig. 4). These results indicate that PDX-1 is involved in the expression of the K_{ATP} channels and the VDCCs.

### [Induction by NeuroD of Expression of Genes Associated with the β-Cell Phenotype]

The present inventors introduced NeuroD into mouse fetal hepatocytes by means of the adenovirus system (Fig. 4). Overexpression of NeuroD did not significantly increase the expression levels of the insulin genes. The expression of the Kir6.2 gene was unaffected by NeuroD, and that of the SUR 1 gene was significantly increased by overexpression of NeuroD (Fig. 4). The expression of the α₁1.3 subunit gene also was increased by overexpression of NeuroD (Fig. 4). These results indicate that NeuroD is involved in the expression of both the K_{ATP} channel and VDCC genes.

Both PC2 and PC1/3 are expressed in the brain and neuroendocrine cells and endocrine cells [Steiner DF, Curr Opin Chem Biol 2:31-39 (1998)]. Thus, the expression of these convertases reflects the characteristics of neuronal, endocrine and neuroendocrine cells. Though the expression levels of both PC2 and PC1/3 were very low in the control hepatocytes, overexpression of NeuroD dramatically increased the expression of the PC1/3 gene (Fig. 4).

Nicotinamide not only prevents β-cell damage due to the activation of poly(ADP-ribose) synthetase/polymerase (PARP) [Yamamoto H et al., Nature 294:284-286(1981)], but also induces endocrine differentiation in cultured human fetal pancreatic islet cells and an increase in insulin gene expression [Otonkoski T et al., J Clin Invest 92:1459-1466 (1993)]. The results shown in the present example indicate that a high concentration of nicotinamide is critical in induction of differentiation of mouse fetal hepatocytes into insulin-producing cells.

The present inventors attempted to generate cells with the pancreatic β-cell phenotype from mouse fetal hepatocytes, which abundantly contain progenitor cells [Dabeva MD et al., Am J Pathol 156:2017-2031 (2000)]. The present inventors found that insulin-producing cells can be induced in an *in vitro* culture system of primitive hepatic progenitor cells [Suzuki A et al., Hepatology 32:1230-1239 (2000)] with a high concentration of nicotinamide as the crucial factor. In addition, it was also found that several genes associated with the pancreatic β-cell phenotype were expressed in fetal hepatocytes under the conditions employed and that their expressions were induced by overexpression of PDX-1 or NeuroD by adenovirus-mediated gene transfer system. These findings indicate that mouse fetal hepatocytes contain progenitor cells that can differentiate *in vitro* into cells with β-cell-like phenotype. Therefore, hepatic progenitor cells can provide a source for generation of transplantable insulin-producing cells.

The homeodomain transcription factor PDX-1, which is necessary in the development of the pancreas, regulates the expression of genes involved in glucose responsiveness as well as the insulin gene [Edlund H, Diabetes 47:1817-1823 (1998), Watada H et al., Diabetes 45:1478-1488 (1996)]. However, although insulin, GLUT2 and glucokinase were all expressed in the mock-infected fetal hepatocytes, overexpression of PDX-1 did not significantly increase the expression of insulin, GLUT2 or glucokinase under the culture condition employed in the present study. The above finding of the high level expression of GLUT2 gene in fetal hepatocytes confirms a previous report [Postic C et al., Am J Physiol 266:E548-559 (1994)]. While glucokinase is expressed predominantly in the adult liver, type I hexokinase is expressed in the fetal liver [Postic C et al., Am J Physiol 266:E548-559 (1994)]. Although we demonstrated that expression of the glucokinase gene is induced in a culture system of primitive hepatic progenitor cells, its mechanism is unknown.

The basic helix-loop-helix (bHLH) transcription factor NeuroD is important in neuronal differentiation as well as in pancreatic development [Edlund H, Diabetes 47:1817-1823 (1998)]. As pancreatic β-cells and neurons share such features as electrical excitability and regulated exocytosis, the present inventors hypothesized that NeuroD might well induce expression of the genes involved in such function. Actually, the expression levels of K_{ATP} channel SUR1 subunit and the VDCC subunit a₁1.3 gene, both of which are expressed in neurons and pancreatic β-cells [Miki T et al., Nat Neurosci 4:507-512 (2001), Seino S et al., Proc Natl Acad Sci USA 89:584-588 (1992)], were increased in fetal hepatocytes by overexpression of NeuroD. PC2 and P1/3 are the principal convertases in regulated secretion in neuroendocrine cells [Steiner DF, Curr Opin Chem Biol 2:31-39 (1998)]. As found by the present inventors, NeuroD markedly induced the expression of PC 1/3, which can cleave proinsulin to yield insulin, indicating that the expression of NeuroD is required for the processing of proinsulin.

## Claims

1. A method for preparation of insulin-producing cells from non-insulin-producing cells, wherein the non-insulin-producing cells are non-human mammalian fetal hepatocytes, and wherein the method comprises culturing the non-human mammalian fetal hepatocytes with 1-50 mmol/L of nicotinamide and concurrently bringing about expression of the NeuroD gene in the non-human mammalian fetal hepatocytes, wherein the expression of the gene is achieved through introduction of the gene into the non-human, mammalian fetal hepatocytes.

2. A method for preparation of insulin-producing cells from non-insulin-producing cells taken from non-fetal mammalian liver, wherein the non-insulin-producing cells are mammalian hepatic progenitor cells, and wherein the method comprises culturing the mammalian hepatic progenitor cells with 1-50 mmol/L of nicotinamide and concurrently bringing about expression of the NeuroD gene in the mammalian hepatic progenitor cells, wherein the expression of the gene is achieved through introduction of the gene into the mammalian hepatic progenitor cells.

3. The method of claim 2, wherein the mammalian hepatic progenitor cells are non-human, mammalian hepatic progenitor cells.

4. A method for causing transdifferentiation of non-human mammalian fetal hepatocytes into insulin-producing cells, wherein the method comprises culturing the non-human mammalian fetal hepatocytes with 1-50 mmol/L of nicotinamide and bringing about expression of the NeuroD gene in the non-human mammalian fetal hepatocytes, wherein the expression of the gene is achieved through introduction of the gene into the non-human mammalian fetal hepatocytes.

5. A method for causing transdifferentiation of non-fetal mammalian hepatic progenitor cells into insulin-producing cells, wherein the method comprises culturing the non-fetal mammalian hepatic progenitor cells with 1-50 mmol/L of nicotinamide and bringing about expression of the NeuroD gene in the non-fetal mammalian hepatic progenitor cells, wherein the expression of the gene is achieved through introduction of the gene into the non-fetal mammalian hepatic progenitor cells.

6. The method of claim 5, wherein the mammalian hepatic progenitor cells are non-human, mammalian hepatic progenitor cells.

7. Non-human mammalian fetal hepatocyte-derived, NeuroD gene-expressing insulin-producing cells prepared by a method comprising culturing non-human mammalian fetal hepatocytes with 1-50 mmol/L of nicotinamide and concurrently bringing about expression of the NeuroD gene in the non-human mammalian fetal hepatocytes, wherein the expression of the gene is achieved through introduction of the gene into the non-human mammalian fetal hepatocytes.

8. Non-fetal mammalian hepatic progenitor cell-derived, NeuroD gene expressing insulin-producing cells prepared by a method comprising culturing non-fetal mammalian hepatic progenitor cells with 1-50 mmol/L of nicotinamide and concurrently bringing about expression of the NeuroD gene in the mammalian hepatic progenitor cells, wherein the expression of the gene is achieved through introduction of the gene into the mammalian hepatic progenitor cells.

9. The cells according to claim 8, wherein the non-fetal mammalian hepatic progenitor cells are non-human.

## Patentansprüche

1. Verfahren zur Herstellung von Insulin-produzierenden Zellen aus nicht-Insulin-produzierenden Zellen, wobei die nicht-Insulin-produzierenden Zellen nicht-humane, fötale Säugetierhepatozyten sind und wobei das Verfahren das Kultivieren der nicht-humanen, fötalen Säugetierhepatozyten mit 1-50 mmol/l Nicotinamid und das gleichzeitige Veranlassen der Expression des NeuroD-Gens in den nicht-humanen, fötalen Säugetierhepatozyten, wobei die Expression des Gens durch Einschleusen des Gens in die nicht-humanen, fötalen Säugetierhepatozyten erreicht wird, umfasst.

2. Verfahren zur Herstellung von Insulin-produzierenden Zellen aus nicht-Insulin-produzierenden Zellen, die aus nicht-fötaler Säugetierleber entnommen wurden, wobei die nicht-Insulin-produzierenden Zellen hepatische Säugetier-Progenitorzellen sind und wobei das Verfahren das Kultivieren der hepatischen Säugetier-Progenitorzellen mit 1-50 mmol/l Nicotinamid und das gleichzeitige Veranlassen der Expression des NeuroD-Gens in den hepatischen Säugetier-Progenitorzellen, wobei die Expression des Gens durch Einschleusen des Gens in die hepatischen Säugetier-Progenitorzellen erreicht wird, umfasst.

3. Verfahren nach Anspruch 2, wobei die hepatischen Säugetier-Progenitorzellen nicht-humane, hepatische Säugetier-Progenitorzellen sind.

4. Verfahren zum Bewirken einer Transdifferenzierung nicht-humaner, fötaler Säugetierhepatozyten in Insulin-produzierenden Zellen, wobei das Verfahren das Kultivieren der nicht-humanen, fötalen Säugetierhepatozyten mit 1-50 mmol/l Nicotinamid und das Veranlassen der Expression des NeuroD-Gens in den nicht-humanen, fötalen Säugetierhepatozyten, wobei die Expression des Gens durch Einschleusen des Gens in die nicht-humanen, fötalen Säugetierhepatozyten erreicht wird, umfasst.

5. Verfahren zum Bewirken einer Transdifferenzierung nicht-fötaler, hepatischer Säugetier-Progenitorzellen in Insulin-produzierende Zellen, wobei das Verfahren das Kultivieren der nicht-fötalen, hepatischen Säugetier-Progenitorzellen mit 1-50 mmol/l Nicotinamid und das Veranlassen der Expression des NeuroD-Gens in den nicht-fötalen, hepatischen Säugetier-Progenitorzellen, wobei die Expression des Gens durch Einschleusen des Gens in die nicht-fötalen, hepatischen Säugetier-Progenitorzellen erreicht wird, umfasst.

6. Verfahren nach Anspruch 5, wobei die hepatischen Säugetier-Progenitorzellen nicht-humane, hepatische Säugetier-Progenitorzellen sind.

7. Nicht-humane, fötale, von Hepatozyten stammende, NeuroD-Gen exprimierende, Insulin produzierende Säugetierzellen, hergestellt mit einem Verfahren, das das Kultivieren nicht-humaner, fötaler Säugetierhepatozyten mit 1-50 mmol/l Nicotinamid und das gleichzeitige Veranlassen der Expression des NeuroD-Gens in den nicht-humanen, fötalen Säugetierhepatozyten, wobei die Expression des Gens durch Einschleusen des Gens in die nicht-humanen, fötalen Säugetierhepatozyten erreicht wird, umfasst.

8. Nicht-fötale, hepatische, von Progenitorzellen stammende, NeuroD-Gen exprimierende, Insulin produzierende Säugetierzellen, hergestellt mit einem Verfahren, das das Kultivieren nicht-fötaler, hepatischer Säugetier-Progenitorzellen mit 1-50 mmol/l Nicotinamid und das gleichzeitige Veranlassen der Expression des NeuroD-Gens in den hepatischen Säugetier-Progenitorzellen, wobei die Expression des Gens durch Einschleusen des Gens in die hepatischen Säugetier-Progenitorzellen erreicht, wird, umfasst.

9. Zellen nach Anspruch 8, wobei die nicht-fötalen, hepatischen Säugetier-Progenitorzellen nicht-human sind.

## Revendications

1. Procédé pour la préparation de cellules productrices d'insuline à partir de cellules non productrices d'insuline, dans lequel les cellules non productrices d'insuline sont des hépatocytes foetaux de mammifère non humain, et dans lequel le procédé comprend la culture des hépatocytes foetaux de mammifère non humain avec 1 à 50 mmoles/l de nicotinamide et l'induction simultanément de l'expression du gène NeuroD dans les hépatocytes foetaux de mammifère non humain, dans lequel l'expression du gène est obtenu par l'introduction du gène dans les hépatocytes foetaux de mammifère non humain.

2. Procédé pour la préparation de cellules productrices d'insuline à partir de cellules non productrices d'insuline prélevées du foie de mammifère non foetal, dans lequel les cellules non productrice d'insuline sont des cellules précurseurs hépatiques de mammifère, et dans lequel le procédé comprend la culture des cellules précurseurs hépatiques de mammifère avec 1 à 50 mmoles/l de nicotinamide et l'induction simultanément de l'expression du gène NeuroD dans les cellules précurseurs hépatiques de mammifère, dans lequel l'expression du gène est obtenue par l'introduction du gène dans les cellules précurseurs hépatiques de mammifère.

3. Procédé selon la revendication 2, dans lequel les cellules précurseurs hépatiques de mammifère sont des cellules précurseurs hépatiques de mammifère non humaines.

4. Procédé pour entraîner la transdifférenciation d'hépatocytes foetaux de mammifère non humain en cellules productrices d'insuline, dans lequel le procédé comprend la culture des hépatocytes foetaux de mammifère non humain avec 1 à 50 mmoles/l de nicotamide et l'induction de l'expression du gène NeuroD dans les hépatocytes foetaux de mammifère non humain, dans lequel l'expression du gène est obtenue par l'introduction du gène dans les hépatocytes foetaux de mammifère non humain.

5. Procédé pour induire la transdifférenciation de cellules précurseurs hépatiques de mammifère non foetales en cellules productrices d'insuline, dans lequel le procédé comprend la culture des cellules précurseurs hépatiques de mammifère non foetales avec 1 à 50 mmoles/l de nicotinamide et l'induction de l'expression du gène NeuroD dans les cellules précurseurs hépatiques de mammifère non foetales, dans lequel l'expression du gène est obtenue par l'introduction du gène dans les cellules précurseurs hépatiques de mammifère foetales.

6. Procédé selon la revendication 5, dans lequel les cellules précurseurs hépatiques de mammifère sont des cellule précurseurs hépatiques de mammifère non humaines.

7. Cellules productrices d'insuline exprimant le gène NeuroD dérivées d'hépatocytes foetaux de mammifère non humain préparées par un procédé comprenant la culture des hépatocytes foetaux de mammifère non humain avec 1 à 50 mmoles/l de nicotinamide et l'induction simultanément de l'expression du gène NeuroD dans les hépatocytes foetaux de mammifère non humain, dans lequel l'expression du gène est obtenue par l'introduction du gène dans les hépatocytes foetaux de mammifère non humain.

8. Cellules productrices d'insuline exprimant le gène NeuroD dérivées de cellules précurseurs hépatiques de mammifère non humain, préparées par un procédé comprenant la culture de cellules précurseurs hépatiques de mammifère non humain avec 1 à 50 mmoles/l et l'induction simultanément de l'expression du gène NeuroD dans les cellule précurseurs hépatiques de mammifère, dans lequel l'expression du gène est obtenue par l'introduction du gène dans les cellules précurseurs hépatiques de mammifère.

9. Cellules selon la revendication 8, dans lesquelles les cellules précurseurs hépatiques de mammifère non foetales sont non humaines.


### SEQUENCE LISTING

<110> Susumu Seino, JCR Pharmaceuticals Co., Ltd.
<120> Induction of insulin-producing cells
<130> GP55
<150> JP2002-115064
   <151> 2002-04-17
<160> 27
<210> 1
   <211> 22
   <212> DNA
   <213> Mus musculus
<400> 1
   tagtgaccag ctataatcag ag 22
<210> 2
   <211> 20
   <212> DNA
   <213> Mus musculus
<400> 2
   acgccaaggt ctgaaggtcc 20
<210> 3
   <211> 19
   <212> DNA
   <213> Mus musculus
<400> 3
   ccctgctggc cctgctctt 19
<210> 4
   <211> 20
   <212> DNA
   <213> Mus musculus
<400> 4
   aggtctgaag gtcacctgct 20
<210> 5
   <211> 18
   <212> DNA
   <213> Mus musculus
<400> 5
   tgagttcctt ccagttcg 18
<210> 6
   <211> 19
   <212> DNA
   <213> Mus musculus
<400> 6
   agggagctgg tgttgtgta 19
<210> 7
   <211> 20
   <212> DNA
   <213> Mus musculus
<400> 7
   cagaagggaa caacatcgtg 20
<210> 8
   <211> 20
   <212> DNA
   <213> Mus musculus
<400> 8
   ccgccaatga tcttttcgta 20
<210> 9
   <211> 20
   <212> DNA
   <213> Mus musculus
<400> 9
   tgaagaagaa gctgcggtca 20
<210> 10
   <211> 20
   <212> DNA
   <213> Mus musculus
<400> 10
   tacatcgtga cccacacagt 20
<210> 11
   <211> 20
   <212> DNA
   <213> Mus musculus
<400> 11
   taggccaagc cagtgtagtg 20
<210> 12
   <211> 20
   <212> DNA
   <213> Mus musculus
<400> 12
   gtggtgaaca catcctgcag 20
<210> 13
   <211> 20
   <212> DNA
   <213> Mus musculus
<400> 13
   ggatgaatgc cttcatcaag 20
<210> 14
   <211> 20
   <212> DNA
   <213> Mus musculus
<400> 14
   ggaagacgtg gttctccttc 20
<210> 15
   <211> 21
   <212> DNA
   <213> Mus musculus
<400> 15
   cttcgtcatc gtcaccttcc a 21
<210> 16
   <211> 20
   <212> DNA
   <213> Mus musculus
<400> 16
   tgaacatctt ggactgctca 20
<210> 17
   <211> 20
   <212> DNA
   <213> Mus musculus
<400> 17
   atggagcaaa gaggttggac 20
<210> 18
   <211> 20
   <212> DNA
   <213> Mus musculus
<400> 18
   gctgcagtca ttctggtatc 20
<210> 19
   <211> 20
   <212> DNA
   <213> Mus musculus
<400> 19
   tcgccaagtt gcagcagaac 20
<210> 20
   <211> 21
   <212> DNA
   <213> Mus musculus
<400> 20
   cttcggccac gttcaagtct a 20
<210> 21
   <211> 20
   <212> DNA
   <213> Mus musculus
<400> 21
   cagatcggca atgcctgctg 20
<210> 22
   <211> 20
   <212> DNA
   <213> Mus musculus
<400> 22
   gaggtgaagc cagagccagt 20
<210> 23
   <211> 20
   <212> DNA
   <213> Mus musculus
<400> 23
   ctaaggcctg gcttgtagct 20
<210> 24
   <211> 20
   <212> DNA
   <213> Mus musculus
<400> 24
   cggctatcca actggctctc 20
<210> 25
   <211> 1049
   <212> DNA
   <213> Mus musculus
<400> 25
<210> 26
   <211> 1211
   <212> DNA
   <213> Homo sapiens
<400> 26
   tttagggagc ggaagctgaa ggcgtatctg gcttttgaat atagcgtttt tctgcttttc      60
   tttctgttcg cctctccctt gttgaatgta ggaaatcgaa acatgaccaa atcgtacagc     120
   gagagtgggc tgatgggcga gcctcagccc caaggtcctc caagctggac agacgagtgt     180
   ctcagttccc aggacgagga gcacgaggca gacaagaagg aggacgacct cgaagccatg     240
   aacgcagagg aggactcact gaggaacggg ggagaggagg aggacgaaga tgaggacctg     300
   gaagaggagg aagaagagga agaggaggat gacgatcaaa agcccaagag acgcggcccc     360
   aaaaagaaga agatgactaa ggctcgcctg gagcgtttta aattgagacg catgaaggct     420
   aacgcccggg agcggaaccg catgcacgga ctgaacgcgg cgctagacaa cctgcgcaag     480
   gtggtgcccc gctattctaa gacgcagaag ctgtccaaaa tcgagactct gcgcttggcc     540
   aagaactaca tctgggctct gtcggagatc ctgcgctcag gcaaaagccc agacctggtc     600
   tccttcgtcc agacgctttg caagggctta tcccaaccca ccaccaacct ggttgcgggc     660
   tgcctgcaac tcaatcctcg gacttttctg cctgagcaga accaggacat gcccccccac     720
   ctgccgacgg ccagcgcttc cttccctgta cacccctact cctaccagtc gcctgggctg     780
   cccagtccgc cttacggtac catggacagc tcccatgtct tccacgttaa gcctccgccg     840
   cacgcctaca gcgcagcgct ggagcccttc tttgaaagcc ctctgactga ttgcaccagc     900
   ccttccttcg atggacccct cagcccgccg ctcagcatca atggcaactt ctctttcaaa     960
   cacgaaccgc ccgcccagtt tgagaaaaat tatgccttta ccatgcacta tcctgcagcg    1020
   acactggcag gggcccaaag ccacggatca atcttctcag gcaccgctgc ccctcgctgc    1080
   gagatcccca tagacaatat tatgtccttc gatagccatt cacatcatga gcgagtcatg    1140
   agtgcccagc tcaatgccat atttcatgat tagaggcacg ccagtttcac catttccggg    1200
   aaacgaaccc a                                                         1211
<210> 27
   <211> 1099
   <212> DNA
   <213> Homo sapiens
<400> 27
   ggaaatcgaa acatgaccaa atcgtacagc gagagtgggc tgatgggcga gcctcagccc 60
   caaggtcctc caagctggac agacgagtgt ctcagttctc aggacgagga gcacgaggca 120
   gacaagaagg aggacgacct cgaagccatg aacgcagagg aggactcact gaggaacggg 180
   ggagaggagg aggacgaaga tgaggacctg gaagaggagg aagaagagga agaggaggat 240
   gacgatcaaa agcccaagag acgcggcccc aaaaagaaga agatgactaa ggctcgcctg 300
   gagcgtttta aattgagacg catgaaggct aacgcccggg agcggaaccg catgcacgga 360
   ctgaacgcgg cgctagacaa cctgcgcaag gtggtgcctt gctattctaa gacgcagaag 420
   ctgtccaaaa tcgagactct gcgcttggcc aagaactaca tctgggctct gtcggagatc 480
   ctgcgctcag gcaaaagccc agacctggtc tccttcgttc agacgctttg caagggctta 540
   tcccaaccca ccaccaacct ggttgcgggc tgcctgcaac tcaatcctcg gacttttctg 600
   cctgagcaga accaggacat gcccccccac ctgccgacgg ccagcgcttc cttccctgta 660
   cacccctact cctaccagtc gcctgggctg cccagtccgc cttacggtac catggacagc 720
   tcccatgtct tccacgttaa gcctccgccg cacgcctaca gcgcagcgct ggagcccttc 780
   tttgaaagcc ctctgactga ttgcaccagc ccttcctttg atggacccct cagcccgccg 840
   ctcagcatca atggcaactt ctctttcaaa cacgaaccgt ccgccgagtt tgagaaaaat 900
   tatgccttta ccatgcacta tcctgcagcg acactggcag gggcccaaag ccacggatca 960
   atcttctcag gcaccgctgc ccctcgctgc gagatcccca tagacaatat tatgtccttc 1020
   gatagccatt cacatcatga gcgagtcatg agtgcccagc tcaatgccat atttcatgat 1080
   tagaggcacg ccagtttca 1099